# EUROPEAN PATENT APPLICATION

(11) **EP 1 808 178 A1**
(43) Date of publication of application: **18.07.2007**
(21) Application number: 05026926.5
(22) Date of filing: 09.12.2005
(51) Int. Cl.: A61K 36/73, A61P 35/00

(54) **Composition comprising Astragalus radix extracts for treating prostate cancer**

(71) Applicant: Golden Biotechnology Corp., Danshuei Township Taipei County (TW)
(72) Inventor: Wang, Yu Lung, 48 Regent Street Cambridge GB2 1FD (GB); Liu, Sheng Yung, 48 Regent Street Cambridge GB2 1FD (GB); Huang, San Bao, 48 Regent Street Cambridge GB2 1FD (GB)
(74) Representative: Horak, Michael

(57) **Abstract**

A composition for effectively suppressing the growth of Prostate Cancer Cell, suppressing the Prostatic Hyperplasia and its preparation method, wherein this composition contains the Astragalusradix or its extracts with effective ingredients, which can either prevent or cure the Prostate cancer and suppress the Prostatic Hyperplasia as well.

## Description

### BACKGROUND OF THE INVENTION

### (a) Technical Field of the Invention

The present invention relates generally to a kind of composition for effectively suppressing the growth of Prostate Cancer Cell, suppressing the Prostatic Hyperplasia and its preparation method.

### (b) Description of the Prior Art

The prostate cancer is a critical male cancer and even prostate cancer is the second leading cause of death among men in the western countries. In our country, the incidence rate of prostate cancer has been increasing year by year substantially

According to the reports submitted by the National Cancer Institute of American, people who eat soybean products can reduce the incidence rate for prostate cancer. In light of the investigations, it shows that patient numbers relating to prostate cancer diseases in East Asia region is much less than Euro-American region, and the main reason disclosed might be involved in the possibility of daily digestible value of soybean (with effective composition of Soy Isoflavones) (Note 1), and many researches have disclosed that Soy Isoflavones is capable to reduce the incidence rate of prostate cancer (Note 2), in addition, the prostate cancer research projects held by the Harvard Medical School of American, had also confirmed that Soy Isoflavones can indeed suppress the growth of prostate cancer by the verification of animal experiments (Note 3).

Prostate, containing prostate cells (PZ-HPV-7 cells), which is a part of male reproductive system, locating at the urinary bladder-outlet and wrapping around the proximal urethra, most of male over 50 years old, almost have a Prostatism in a different level (due to human prostate cells (PZ-HPV-7 cells) containing the epidermal growth factor), which is a normal symptom, and thus results in the following effects:
1) Decreasing force of urination, it's difficult to start urine flow immediately.
2) Urinary frequency and noturia: Increasing urination times, getting up frequently to urinate at midnight.
3) Residual urine: still dribble a little urine after finished urinating.
4) Acute bladder hypotonicity: difficult to urinate suddenly while bladder is full.
5) Hematuria.

Other symptoms including:
Urinary tract infection: showing symptoms of frequent urination, having to pass urine suddenly or often, burning or pain while passing urine etc., and finding out pus cells and germs during cystourethroscopy inspection.
Bladder stone: causing pain during urination or sudden interruption of urinary stream.
Bladder Diverticulum: Bladder diverticula are herniations of the bladder mucosa through the bladder wall musculature, therefore it is necessary to have urination twice, due to the urine inside bladder is impossible to be passed out completely once, therefore needs to urinate the urine again from additional Diverticulums.
Urine overflow incontinence: overflow in bladder, and incontinence happened while pressure is exceeding the urethra resistance and pass urine out of control.
No matter the prostate cancer or the Prostatic Hyperplasia etc., it will cause a huge burden to national health insurance system in addition to the tremendous threat or perplexing to personal life quality, and thus it is the obligation for the medicine, pharmacy industry without any doubt to find out more effective drugs.

The applications of Astragalusradix had been recorded in various Traditional Chinese Medicine and Pharmacopoeia, according to «Chinese Bencao» (Shanghai Science and Technology Publishing House, 1999), based on the relevant researches in Mainland China during recent years, the Astragalusradix has various effects such as: to enhance the immunity function, improve the activity of killer cells, enhance the resistance to diseases, anti-aging, anti-oxidation and nutritional anemia treatment, improve the cardiovascular system, multiple virus resistance, and anti-cancers etc. Regarding the anti-cancers effect, it has been found out during animal experiments that the Astragalusradix is capable to reduce the occurrence rate of the big rats on lung cancers, to extend the lifetime of small rats suffered from melanoma, and there is at least one additional successful case for stomach cancer patient and two successful cases for ovarian cancer patients during clinical experiments, in which the cancer cells were killed by taking polysaccharides extract of Radix Astragali.

In light of the medical papers in the western countries, the Astragalusradix in vitro experiments shown that it has a effect in suppressing the stomach cancer cells (Note 4), and the result disclosed in the animal experiment, the Astragalusradix extracts are capable to effectively decrease the incidence rate of urinary bladder tumors caused by the carcinogen N-butyl-N-the butanolnitrosoamine (Note 5), while taking together with other Chinese herbal medicines, it showed that the TCM Prescription containing Radix Angelicae Sinensis and Radix Astragali has suppressed cancer cells transformed underneath mice skins (Note 6), and the composition of Juzen-taiho-to which contains Cinnamate, Radix Astragali and another two kinds of Japanese herb medicines has a effect in suppressing the liver cells transformed by colorectal cancer cells as well as lung cells transformed by melanoma accordingly (Note 7), in addition, when TCM Prescription containing 10 different Chinese herb medicines including Radix Astragali to be used together with mitomycin C in order to treat mice having an implantation of blood cancer cells, it showed that the lifetime of these mice is longer than other mice having treatment of mitomycin C only (Note 8), in light of the above information, we found out that Radix Astragali has been utilized popularly in various experiments for cancer treatments, however until this moment, there is neither any research report related to the application of Radix Astragali on the treatment of prostate cancer nor any effects on suppressing Human's Prostatism as well.
Note 1 - Yun ' T.K.1999.Ann.NYAcad.Sci. ' 889:157-92;Persky ' V.andI.Van Hom.1995.J.Nutr. ' 125(3):709-712S;Messina ' M. ' et.al. ' 1994.Nutr.Cancer ' 21:113-131;Haytowitz ' D.B.1995J.Nutr. ' 125:1952-1955
Note 2 - Messina ' M.J.2003.Nutr.Rev. ' 61(4):117-31; Zhou ' J.R.et.al. ' Prostate ' 53:143-153.
Note 3 - Prostate ' 2002 ' 53:143-153.
Note 4 - Linet.al. ' WorldJGastroentero1.2003.9:670.
Note 5 - Kurashigeet.al. ' CancerInvest.1999.17:30.
Note 6 - Hsiehet.al. ' ImmunopharmacolImmunotoxicol.2003.25:259.
Note 7 - Onishiet.al. ' BiolPharmBull.1998.21:761.
Note 8 - Aburadaet.al. ' JPharmacobiodyn.1983.6:1000

### SUMMARY OF THE INVENTION

The primary purpose of the present invention is find out the effects for Astragalusradix extracts, which consists of four major ingredients, namely (1) Aminoacid (major ingredient of protein), (2) Polysaccharides (having a more tightening combination for Aminoacid), (3) Saponins (anti-inflammation), (4) Flavonoids (increasing estrogen to suppress androgen), and its compositions are capable to suppress the prostate cancer as well as suppress the Prostatic Hyperplasia, and in accordance with a series of experiments, in vitro, it has been verified that its effects is even much more effective than Soy Isoflavones. The present invention has verified that the Astragalusradix extracts, or a new composition of PCF 1, which consists of effective extracted ingredients of Astragalusradix and Soy Isoflavones etc., and it shows that both effects of Astragalusradix extracts and new complex composition are capable to suppress both the growth of Prostate Cancer Cell and also the symptom of Prostatic Hyperplasia at the same time.

The foregoing object and summary provide only a brief introduction to the present invention. To fully appreciate these and other objects of the present invention as well as the invention itself, all of which will become apparent to those skilled in the art, the following detailed description of the invention and the claims should be read in conjunction with the accompanying drawings. Throughout the specification and drawings identical reference numerals refer to identical or similar parts.

Many other advantages and features of the present invention will become manifest to those versed in the art upon making reference to the detailed description and the accompanying sheets of drawings in which a preferred structural embodiment incorporating the principles of the present invention is shown by way of illustrative example.

### BRIEF DESCRIPTION OF THE DRAWINGS

Figure 1 is the high-performance liquid chromatographic profile of PCF1;
Figure 2 is shows the comparable growth-suppressing capability against the human prostate cancer LNCaP cell lines among the Soy Isoflavones (control group), Astragalusradix extracts and PCF 1;
Figure 3 shows the growth-suppressing capability of PCF 1 against the human prostate cancer LNCaP cell lines in different composition ratio;
Figure 4 shows the capability of human Leukocyte cells to release TNF-α which is activated by PCF1;
Figure 5 shows the capability of human Leukocyte cells to release IFN-γ which is activated by PCF 1;
Figure 6 shows the PCF 1 is suppressing the growth of prostate cells PZ-HPV-7;
Table 1 shows the growth-suppressing capability of PCF1 against the human prostate cancer LNCaP cell lines in different composition ratio;
Table 2 shows the growth-suppressing capability ofTaxol against the human prostate cancer LNCaP cell lines with the assistance provided by PCF1;
Table 3 shows the suppressing capability of PCF1 against the blood levels of prostate specific antigen (PSA) and the growth of the prostate cancer tumor; and
Table 4 shows the PCF 1 is suppressing the growth of prostate cells PZ-HPV-7.

### DETAILED DESCRIPTION OF THE PREFERRED EMBODIMENTS

The following descriptions are of exemplary embodiments only, and are not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention as set forth in the appended claims.

Production example: The production of Astragalusradix extracts and composition containing Astragalusradix extracts:

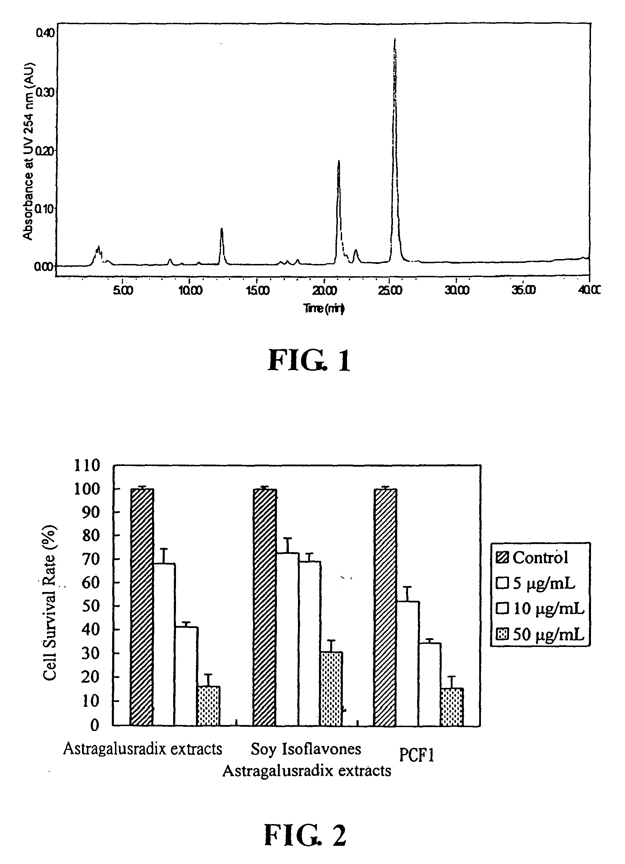

The Astragalusradix extracts, as cited by the general arts, can be made by grinding 100g wild original Astragalusradix root material into small pieces, and then adding 2L water, heating it until boiling point for recirculation about 1-4 hours, and then 30% Astragalusradix extract would be acquired by filtering, concentrating and drying the extractive solution; where the Soy Isoflavones containing 20% Aglycone Form which is bought from the market; and the new complex composition - PCF 1, consisting of Astragalusradix extract and Soy Isoflavones having the specific gravity for extracts in a proportional ratio of 1:0 to 1:2, where its high-performance liquid chromatographic profile is shown in Figure 1, and the high-performance liquid chromatographic instrument has a column of "ThermoHypersil-KeystoneRPC18", 4.6 × 250mm; mobile phase for the high-performance liquid chromatographic instrument is 35% MeOH in 0 minute, 70% MeOH in 30 minutes, 35% MeOH in 35 minutes, 35% MeOH in 40 minutes, with content of 0.1 %TFA; and the flow speed for the high-performance liquid chromatographic instrument is at 1mL/min; where its detecting device is DAD, and wave length is set at 254nm.

### Experimental Example I: In vitro mode anti-tumor activity assay example:

Based on the anti-tumor drugs filter mode provided by the National Cancer Institute (NCI) in America, utilize in vitro filter mode for human prostate cancer cell lines- LNCaP cells. Where human prostate cancer cell lines - LNCaP cells is being cultured in the culture medium which is supplemented by fetalbovine serum for 24 hours, and then change the culture medium so as to culture it in a new culture medium by adding assay sample for 72 hours, afterwards evaluate the cell survival rate by MIT colorimetric analysis method.

### Experimental Example II: In vitro mode adjunctive treatment activity assay example:

Utilize in vitro filter mode for human prostate cancer cell lines- LNCaP cells. Where human prostate cancer cell lines - LNCaP cells is being cultured in the culture medium which is supplemented by fetalbovine serum for 24 hours, and process cells by Taxol for 72 hours, and then change the culture medium so as to culture it in a new culture medium by adding assay sample for 72 hours, afterwards evaluate the cell survival rate by MIT colorimetric analysis method.

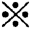 The assay analysis of the compositions in accordance with the present invention in effect for suppressing the growth of human prostate cancer cells, wherein it is necessary to compute the cell survival rate for cells in each well of96-well microplate, afterwards evaluate the cancer cells survival rate by MIT colorimetric analysis method.

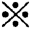 MTT colorimetric analysis method: MTT is a kind of tertrazolium salt, with a full name of 3-[4, 5-Dimenthylthialzol-2-yl] 2, 5-diphenyltetrazoliumbromide, which is the yellow dye, capable to be absorbed by live cells and also be reduced to blue color formazan by succinate-tetrazoliumreductase in mitochondrion, which is commonly used for filtering the affection for matters to the growth and proliferation of cells.

### Experimental Example III: In vitro mode immunomodulation activity assay example:

Utilize in vitro filter mode for human peripheral blood mononuclear cells (MNCs). Where MNCs is being cultured in the culture medium which is supplemented by fetalbovine serum also adding assay samples, such as TNF-α for 24 hours, or adding IFN-γ for 72 hours, and then analyze expression for both TNF-α and IFN-γ by means of TNF-αimmunoassay kit and IFN-γimmunoassay kit.

### Experimental Example IV: In vivo mode anti-tumor activity assay example:

Utilize tumor animal mode for nude rats inoculated the human prostate cancer cell lines-LNCaP cells.

There are 6-weeks old male nude rats (BALB/c-nu/nu) used for the experimental animal mode. Where animals are cut open from abdomen after raised and trained, showing off the prostate, and then inoculating the human prostate cancer cell lines-LNCaP cells (2 x 106cells/50 µ LHBSS/rats) from back side of the prostate with a #30 needle, then use 5-0 threads to sew it up. After a regular feeding for 2 weeks, drawing up blood from each animal for measuring the serum PSA value, which are classified into 4 groups and each group would be processed as the following methods:
Control group: Freely taking the powder meal of rodent chow diet (Purina5010) for 42 days.
PCF1 low-dose group (0.3g/kgbw/day): Freely taking the powder meal of rodent chow diet (Purina5010) containing 0.3g/kgbw/day for 42 days.
PCF1 medium-dose group (1g/kgbw/day): Freely taking the powder meal of rodent chow diet (Purina5010) containing 1g/kgbw/day for 42 days.
PCF1 high-dose group (3g/kgbw/day): Freely taking the powder meal of rodent chow diet (Purina5010) containing 3g/kgbw/day for 42 days.

### Experimental Example V: PCF 1 in vitro mode suppressing the growth activity of human prostate cells assay example:

Utilize in vitro filter mode for human prostate cancer cell lines- LNCaP cells. Where human prostate cancer cell lines-LNCaP cells is being cultured in the Keratinocyte-Serum Free Medium which is supplemented by 5ng/mLepidermal growth factor and bovine pituitary extract for 24 hours, and then change the culture medium so as to culture it in a new culture medium by adding assay sample for 48 hours, afterwards evaluate the cell survival rate by MIT colorimetric analysis method.

### Embodiment I - Astragalusradix extracts anti-tumor activity:

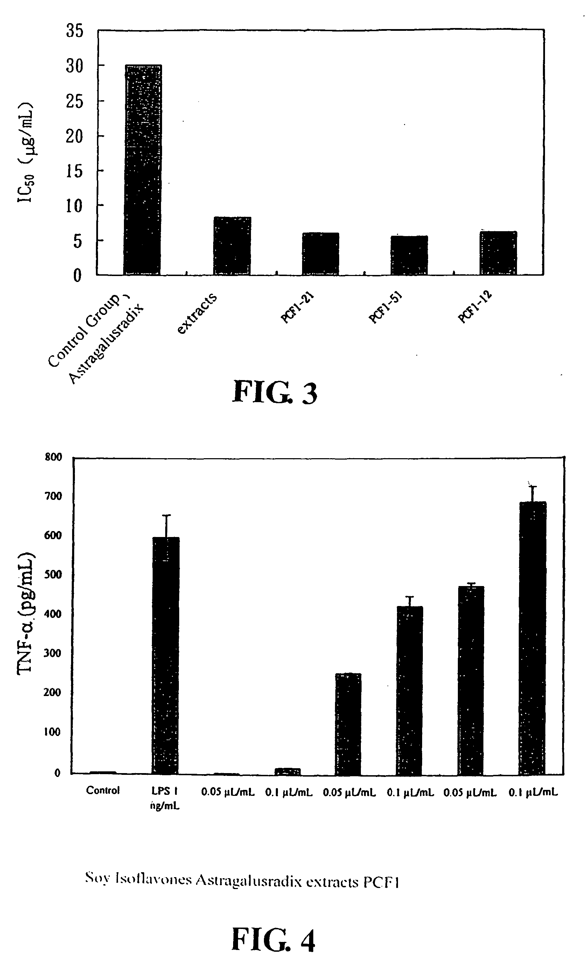

According to the said method of in vitro mode anti-tumor activity assay example, where the concentration (IC50) required for the Astragalusradix extracts to suppress 50% growth rate of the human prostate cancer cells is 8.4µg/mL, and the concentration (IC50) required for the Soy Isoflavones to suppress 50% growth rate of human prostate cancer cells is 30µg/mL, where it is verified that the growth-suppressing effect for the Astragalusradix extracts on human prostate cancer cells is superior to the Soy Isoflavones respectively (as shown in Table 1 and Figure 3).

### Embodiment II - PCF1-21 anti-tumor activity:

According to the said method of in vitro mode anti-tumor activity assay example, while the proportional rate in-between the Astragalusradix extracts to Soy Isoflavones is 2:1, then it shows that the concentration (IC50) required to suppress 50% growth rate of the human prostate cancer cells is 6.1 µg/mL, meaning that the growth-suppressing effect for this composition on human prostate cancer cells is superior to either the Astragalusradix extracts or the Soy Isoflavones individually (as shown in Table 1 and Figure 3).

### Embodiment III - PCF 1-51 anti-tumor activity:

According to the said method of in vitro mode anti-tumor activity assay example, while the proportional rate in-between the Astragalusradix extracts to Soy Isoflavones is 5:1, then it shows that the concentration (IC50) required to suppress 50% growth rate of the human prostate cancer cells is 5.6µg/mL, meaning that the growth-suppressing effect for this composition on human prostate cancer cells is superior to either the Astragalusradix extracts or the Soy Isoflavones individually (as shown in Table 1 and Figure 3).

### Embodiment IV - PCF1-12 anti-tumor activity:

According to the said method of in vitro mode anti-tumor activity assay example, while the proportional rate in-between the Astragalusradix extracts to Soy Isoflavones is 1:2, then it shows that the concentration (IC50) required to suppress 50% growth rate of the human prostate cancer cells is 6.2µg/mL, meaning that the growth-suppressing effect for this composition on human prostate cancer cells is superior to either the Astragalusradix extracts or the Soy Isoflavones individually (as shown in Table 1 and Figure 3).

### Embodiment V - PCF1 adjunctive treatment activity:

According to said method of in vitro mode adjunctive treatment activity assay example, where the 2nMTaxols and 1 µL/mLPCF 1 activating individually, which achieves the cell survival rate of 56.4% and 57.6% for human prostate cancer cells respectively, while it is pre-processed (or post-processed) in conjunction with Taxol and PCF1 respectively, which achieves the cell survival rate of 30.8% for human prostate cancer cells respectively, meaning that PCF 1 is capable to assist the Taxol in suppressing the growth of human prostate cancer cells (as shown in Table 2).

### Embodiment VI - PCF 1 immunomodulation activity:

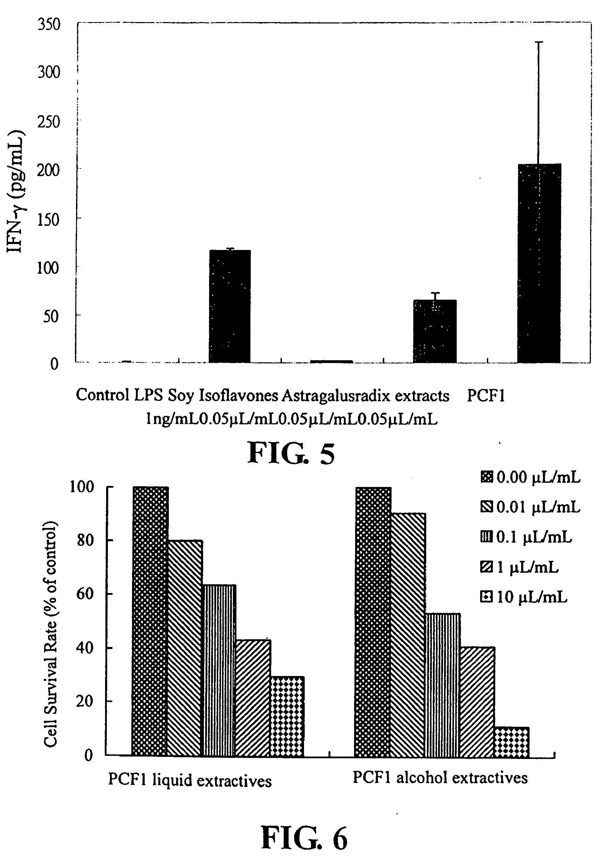

According to said method of in vitro mode immunomodulation activity assay example, where the Astragalusradix has a function for activating the human leukocyte cell to release the TNF-α and IFN-γ, but the Soy Isoflavones has not, and the result showing that PCF 1 is capable to activate the human leukocyte cell to release the TNF-α and IFN-γ which is even superior to the Astragalusradix (as shown in Figure 4, Figure 5).

### Embodiment VII - PCF1 anti-tumor activity:

According to said method of In vivo mode anti-tumor activity assay example, after feeding the animals with PCF1 low-dose (0.3g/kgbw/day) continuously for 42 days, its blood PSA value is 9.1ng/mL which is obviously much lower than that of control group, which is 21.2ng/mL, where the suppressing rate reaches 57%, and the effective growth-suppressing rate for tumor achieves 49% (as shown in Table 3).

### Embodiment VIII - PCF1 suppressing the prostatic hyperplasia activity:

According to said method of in vitro mode activity assay example, it shows that concentration (IC50) required for the extractive solution of PCF1 and alcohol to suppress 50% growth rate of the human prostate cancer cells is 0.019µL/mL and 0.033 µL/mL respectively, meaning that PCF 1 is effectively suppressing the growth of human prostate cells (as shown in Table 4 and Figure 6).

While analyzing the different proportional ratio for each composition in accordance with the present invention resulting in effects in suppressing the growth of cancer cells, we have realized the fact while the proportional weight rate in-between the Astragalusradix to the Soy Isoflavones is in the combination of 1:2-5:1, the composition will have a effective effect in growth-suppressing the human prostate cancer cells obviously, and among them the most superior proportional rate for the said composition is 5:1, as shown in Figure 3, in addition, it has been verified by the animal experiments that the composition of the present invention is capable to effectively suppress the increasing of the blood PSA value as well as the growth of tumors consequently.

The present invention has successfully found out the medicine with Astragalusradix or its composition having an excellent effect in growth-suppressing the prostate cancer cells, where its effects of anti-cancer and cancer prevention have been verified in various theses or reports already, which is much superior to the Soy Isoflavones, and more over, the composition according to the present invention also has an effect in adjunctive treatments which is applicable to assist the anti-cancer drugs such as Taxol in suppressing the growth of prostate cancer cells, and meanwhile it has am additional function of the immunomodulation as well, which is capable of activating the leukocyte cells to release both the TNF-α and IFN-γ accordingly.

In light of the above description, due to the present invention has all the said advantages as well as practical values, where there is no any similar product which has ever been disclosed at all, thus the inventor believes that the present invention shall meet all the requirements for the application of the patent of new invention accordingly.

It will be understood that each of the elements described above, or two or more together may also find a useful application in other types of methods differing from the type described above.

While certain novel features of this invention have been shown and described and are pointed out in the annexed claim, it is not intended to be limited to the details above, since it will be understood that various omissions, modifications, substitutions and changes in the forms and details of the device illustrated and in its operation can be made by those skilled in the art without departing in any way from the spirit of the present invention.

**Table 1**

| | Cell Survival Rate (%) | | | | |
|---|---|---|---|---|---|
| Concentration (µg/mL) | Soy Isoflavones (Control group) | Astragalusradix extracts | PCF1-21 | PCF1-51 | PCF1-12 |
| 5.0 | 73±5 | 68±2 | 52±4 | 52±6 | 52±0 |
| 10 | 69±3 | 41±9 | 41±1 | 35±3 | 45±0 |
| 50 | 31±3 | 16±2 | 7.4±2.8 | 16±7 | 5.4±3.5 |
| IC₅₀(µg/mL) | 30 | 8.4 | 6.1 | 5.6 | 6.2 |

**Table 2**

| | Cell Survival Rate (%) | |
|---|---|---|
| PCF1 Concentration (µL/mL) | PCF1 | Taxol+PCF1 |
| 0.0 | 100±9 | 56±6 |
| 0.1 | 99±3 | 43±0 |
| 1.0 | 58±6 | 31±2 |
| 10 | 18±5 | 14±7 |
| IC₅₀(µL/mL) | 2.7 | 0.049 |

**Table 3**

| Group | | PSA (ng/mL) | Tumor weight (g) |
|---|---|---|---|
| Control n=10 | | 21.2±12.2 | 0.153±0.087 |
| PCF1'0.3g/kg n=10 | | 9.1±8.0* | 0.078±0.063 |
| PCF1 ' 1g/kg n=10 | | 12.9±12.1 | 0.070±0.071* |
| PCF1 ' 3g/kg n=10 | | 14.9±14.6 | 0.093±0.083 |

**Table 4**

| | Cell Survival Rate (% of control) | |
|---|---|---|
| Concentration (µL/mL) | PCF1 liquid extractives | PCF1 alcohol extractives |
| 0.00 | 100 | 100 |
| 0.01 | 80.0 | 90.4 |
| 0.1 | 63.6 | 53.2 |
| 1 | 43.2 | 40.8 |
| 10 | 29.6 | 11.2 |
| IC501 (µL/mL) | 0.019 | 0.033 |
| IC502 (µL/mL) | 12.8 | 4.25 |

## Claims

1. A kind of composition for effectively suppressing the growth of prostate cancer that is **characterized by** the compositions consisting of Leguminosal, Radixettuber and Astragalusradix extracts etc.

2. A kind of composition for effectively suppressing the growth of prostate cancer as claimed in Claim **1**, where the compositions with Astragalusradix extracts containing the content of said Astragalusradix extracts is from 5% to 100% thereof.

3. A kind of composition for effectively suppressing the growth of prostate cancer as claimed in Claim **1**, wherein the said Astragalusradix extracts is water extract.

4. A kind of composition for effectively suppressing the growth of prostate cancer as claimed in Claim **1**, wherein the said Astragalusradix extracts is alcohol extract.

5. A kind of composition for effectively suppressing the growth of prostate cancer as claimed in Claim 1, where the said composition further containing extracts of Astragalusradix and soybean etc.

6. The extracts of Astragalusradix and soybean as claimed in Claim 5, wherein the said soybean extract is Soy Isoflavones.

7. The compositions containing extracts of Astragalusradix and soybean as claimed in Claim 6, wherein the content of said Astragalusradix extracts is from 5% to 100% thereof

8. The compositions containing extracts of Astragalusradix and Soy Isoflavones as claimed in Claim 6, wherein the content ratio for the said Astragalusradix extracts and Soy Isoflavones is 1:1 therefrom.

9. The compositions containing extracts of Astragalusradix and Soy Isoflavones as claimed in Claim 6, wherein the content ratio for the said Astragalusradix extracts and Soy Isoflavones is 1:2 therefrom.

10. The compositions containing extracts of Astragalusradix and Soy Isoflavones as claimed in Claim 6, wherein the content ratio for the said Astragalusradix extracts and Soy Isoflavones is 2:1 therefrom.

11. The compositions containing extracts of Astragalusradix and Soy Isoflavones as claimed in Claim 6, wherein the content ratio for the said Astragalusradix extracts and Soy Isoflavones is 5:1 therefrom.

12. A kind of composition for effectively suppressing the growth of prostate cancer as claimed in Claim 1, which is applicable to support the treatment of prostate cancer.

13. A supporting treatment to prostate cancer as claimed in Claim 12, where the said treatment is the chemotherapy.

14. The chemotherapy as claimed in Claim 13, where the medicine for said chemotherapy is the Taxane type anti-cancer drugs.

15. The Taxane type anti-cancer drugs as claimed in Claim **14**, which is Taxol.

16. A kind of composition for effectively suppressing the growth of prostate cancer as claimed in Claim **1**, which has the effect of immunity regulation.

17. The effect of immunity regulation as claimed in Claim **15,** which is capable to release TNF-α and IFN-γ so as to increase the amount of human peripheral blood mononuclear cells (PBMC).

18. A kind of composition for effectively suppressing the growth of prostate cancer as claimed in Claim **1**, where the treatment receptor is mammal.

19. A kind of composition for effectively suppressing the growth of prostate cells PZ-HPV-7, that is **characterized by** the compositions consisting of Leguminosal, Radixettuber and Astragalusradix extracts etc.

20. A kind of composition for effectively suppressing the growth of prostate cells PZ-HPV-7 as claimed in Claim **19**, where the compositions with Astragalusradix extracts containing the content of said Astragalusradix extracts is from 5% to 100% thereof.

21. A kind of composition for effectively suppressing the growth of prostate cells PZ-HPV-7 as claimed in Claim **19**, wherein the said Astragalusradix extracts is water extract.

22. A kind of composition for effectively suppressing the growth of prostate cells PZ-HPV-7 as claimed in Claim **19**, wherein the said Astragalusradix extracts is alcohol extract.

23. A kind of composition for effectively suppressing the growth of prostate cells PZ-HPV-7 as claimed in Claim **19**, where the treatment receptor is mammal.
